(19) Europäisches Patentamt
European Patent Office
Office européen des brevets

(11) Numéro de publication : **0 359 618 B1**

# (12) FASCICULE DE BREVET EUROPEEN

(45) Date de publication du fascicule du brevet : **17.03.93 Bulletin 93/11**

(51) Int. Cl.$^5$ : **C07C 217/76,** C07C 323/29, A61K 7/13

(21) Numéro de dépôt : **89402372.0**

(22) Date de dépôt : **31.08.89**

## (54) Para-aminophénols 2-substitués et leur utilisation pour la teinture des fibres kératiniques.

Le dossier contient des informations techniques présentées postérieurement au dépôt de la demande et ne figurant pas dans le présent fascicule.

(30) Priorité : **13.09.88 FR 8811926**

(43) Date de publication de la demande : **21.03.90 Bulletin 90/12**

(45) Mention de la délivrance du brevet : **17.03.93 Bulletin 93/11**

(84) Etats contractants désignés : **AT BE CH DE ES FR GB GR IT LI NL SE**

(56) Documents cités :
**EP-A- 0 182 187**
**EP-A- 0 226 072**
**DE-C- 148 977**
**US-A- 3 666 812**
**CHEMICAL ABSTRACTS, vol. 109, no. 15, 10 octobre 1988, page 649, résumé no. 128609s, Columbus, Ohio, US; & JP-A-63 48 256**

(73) Titulaire : **L'OREAL**
**14, Rue Royale**
**F-75008 Paris (FR)**

(72) Inventeur : **Junino, Alex**
**16, rue Docteur Bergonié**
**F-93180 Livry-Gargan (FR)**
Inventeur : **Lang, Gérard**
**44, avenue Lacour**
**F-95210 Saint-Gratien (FR)**
Inventeur : **Genet, Alain**
**9, rue des Coquelicots**
**F-93600 Aulnay-sous-Bois (FR)**

(74) Mandataire : **Casalonga, Axel et al**
**BUREAU D.A. CASALONGA - JOSSE**
**Morassistrasse 8**
**W-8000 München 5 (DE)**

EP 0 359 618 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention a pour objet des para-aminophénols 2-substitués et leur utilisation dans des compositions tinctoriales pour la teinture des fibres kératiniques et en particulier des cheveux humains, ces compositions tinctoriales étant utilisées pour la coloration dite coloration d'oxydation ou teinture permanente.

EP-A-0182 187 enseigne des 4-amino 2-hydroxyalkylphénols utilisables comme précurseurs de colorants d'oxydation dans des compositions tinctoriales pour cheveux. Cependant, la résistance à la lumière des couleurs obtenues n'est pas tout à fait satisfaisante.

On a découvert qu'une nouvelle classe de para-amino-phénols 2-substitués permet d'obtenir sur les fibres kératiniques et en particulier sur les cheveux des colorations d'oxydation ayant une résistance améliorée à la lumière.

Chemical Abstracts, Vol. 109, No. 15, 10 octobre 1988, page 649, résumé No. 128609 s. Columbus, Ohio, US & JP-A-63-48256 décrivent la préparation du 4-amino 2-[(2',2',2'-trifluoroéthoxy)méthyl]phénol.

Cependant, cette référence ne divulgue pas l'utilisation de ce composé pour la teinture des fibres kératiniques.

L'invention a pour objet des composés nouveaux que constituent les para-aminophénols 2-substitués de formule (I) :

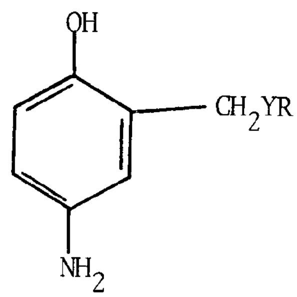

(I)

dans laquelle Y représente un atome d'oxygène ou de soufre et R représente un radical alkyle en $C_1$-$C_6$, hydroxyalkyle en $C_1$-$C_6$, polyhydroxyalkyle en $C_2$-$C_6$ ou halogénoalkyle en $C_1$-$C_6$, avec la condition que lorsque Y désigne un atome d'oxygène, R n'est pas méthyle ou éthyle et lorsque Y désigne un atome de soufre, R n'est pas éthyle, et leurs sels, à l'exclusion du 4-amino 2-[(2',2',2'-trifluoroéthoxy)méthyl]phénol.

L'invention a également pour objet les compositions tinctoriales pour fibres kératiniques et en particulier pour cheveux humains, contenant dans un véhicule aqueux, un ou plusieurs précurseurs de colorants d'oxydation, de formule (I') :

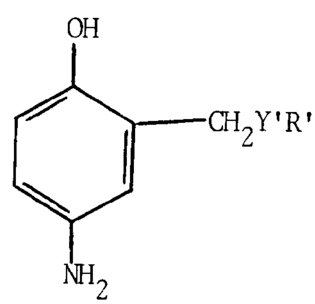

(I')

dans laquelle Y' représente un atome d'oxygène ou de soufre et R' représente un radical alkyle en $C_1$-$C_6$, hydroxyalkyle en $C_1$-$C_6$, polyhydroxyalkyle en $C_2$-$C_6$ ou halogénoalkyle en $C_1$-$C_6$; ou un sel d'un ou plusieurs composés de formule (I'). Les sels sont de préférence des chlorhydrates ou des sulfates.

Les compositions tinctoriales contenant un ou plusieurs composés de formule (I) permettent de conférer aux cheveux, par couplage oxydatif avec d'autres précurseurs de colorants d'oxydation et/ou de coupleurs, des couleurs stables au lavage, à la lumière et aux intempéries. En outre, les para-amino-phénols de formule (I') bénéficient d'une bonne innocuité.

Les composés de formule (I') préférés sont les suivants :

- l'amino-4[(β-hydroxyéthylthio)méthyl]-2 phénol;
- l'amino-4[(méthylthio)méthyl]-2 phénol;
- l'amino-4 méthoxyméthyl-2 phénol (qui peut être préparé selon le procédé décrit dans le brevet allemand 148.977);

- l'amino-4 éthoxyméthyl-2 phénol (qui peut être préparé selon le procédé décrit dans le brevet allemand 148.977);
- l'amino-4 [(β-hydroxyéthoxy)méthyl]-2 phénol;
- l'amino-4 [(β,γ-dihydroxypropylthio)méthyl]-2 phénol;
- l'amino-4 [(trifluoro-2',2',2'éthoxy)méthyl]-2 phénol.

Les composés de formule (I) sont préparés en deux étapes à partir du nitrophénol substitué de formule (III), dans laquelle X désigne un halogène, selon le schéma réactionnel ci-dessous :

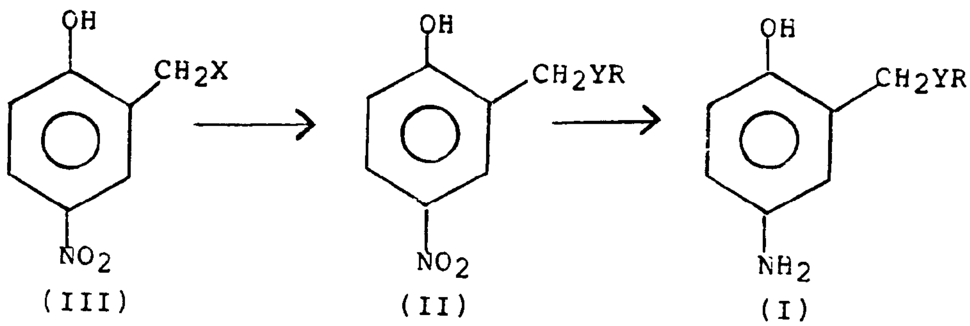

(III) (II) (I)

Le composé de formule (I) est obtenu par réduction du composé de formule (II) où Y et R ont les significations ci-dessus définies. Parmi les méthodes de réduction classiques, on peut citer la réduction par l'hydrosulfite de sodium en milieu alcalin à une température inférieure à 80°C, ou bien une réduction catalytique en milieu hydroalcoolique, sous pression d'hydrogène, en présence d'un catalyseur tel que le Palladium sur charbon ou le Nickel.

Le composé de formule (II) peut être préparé par action d'un alcoolate ou d'un thiolate de formule (IV) :

M - Y - R (IV)

dans laquelle M représente un métal alcalin ou alcalino-terreux et Y et R ont les significations ci-dessus définies.

L'alcoolate et le thiolate de formule (IV) peuvent être avantageusement préparés in situ selon le schéma réactionnel :

$$MOH + HYR \rightarrow MYR + H_2O$$

le composé HYR pouvant également servir de solvant.

Parmi les solvants que l'on peut utiliser pour la préparation des composés de formule (II), on peut citer, outre le composé HYR, le dioxane, le N,N-diméthylformamide, la N-méthylpyrrolidone, utilisés seuls ou en mélange. Généralement, la température de la réaction est inférieure à 100°C.

Les compositions tinctoriales pour fibres kératiniques et en particulier pour cheveux humains, selon l'invention, contiennent, dans un véhicule aqueux, au moins un composé de formule (I').

Les composés de formule (I') sont utilisés dans les compositions de l'invention à des concentrations comprises entre 0,02 et 6% et de préférence comprises entre 0,15 et 5% en poids, par rapport au poids total de la composition.

Les compositions de l'invention peuvent contenir également d'autres précurseurs de colorants par oxydation.

Parmi ces précurseurs de colorants par oxydation, il faut citer les para-phénylènediamines, les para-aminophénols différents de ceux de formule (I'), les ortho-phénylènediamines et les ortho-aminophénols.

Parmi les para-phénylènediamines, il faut citer plus particulièrement :

la para-phénylènediamine,
la para-toluylènediamine,
la diméthyl-2,6 para-phénylènediamine,
la diméthyl-2,6 méthoxy-3 para-phénylènediamine,
la N-(β-méthoxyéthyl)para-phénylènediamine,
la N-[β-(β'-hydroxyéthoxy)éthyl]amino-4 aniline,
la N,N-di(β-hydroxyéthyl)amino-4 aniline,
la N,N-(éthyl,carbamylméthyl)amino-4 aniline,
ainsi que leurs sels.

Parmi les para-aminophénols, il faut citer plus particulièrement :

le para-aminophénol,
le méthyl-2 amino-4 phénol,

le chloro-2 amino-4 phénol,
le diméthyl-2,6 amino-4 phénol,
le diméthyl-2,3 amino-4 phénol,
le diméthyl-2,5 amino-4 phénol,
ainsi que leurs sels.

Parmi les ortho-phénylènediamines et les ortho-aminophénols pouvant être substitués sur le noyau ou sur les fonctions amines, il faut citer en particulier l'amino-1 hydroxy-2 benzène, le méthyl-6 hydroxy-1 amino-2 benzène, le méthyl-4 amino-1 hydroxy-2 benzène.

Les compositions tinctoriales faisant l'objet de la présente invention contiennent généralement en association avec les composés (I') et éventuellement avec des para-phénylènediamines ou avec d'autres para-aminophénols, des coupleurs qui donnent par couplage oxydatif avec les bases d'oxydation des indo-anilines, des indamines ou des indophénols, diversement nuancés qui contribuent à modifier et à enrichir de reflets les colorations "de fond" conférées aux cheveux par les produits de condensation des bases d'oxydation sur elles-mêmes.

Parmi les coupleurs, on peut citer en particulier les méta-diphénols, les méta-aminophénols, les méta-phénylènediamines, les méta-acylaminophénols, les méta-uréidophénols, les méta-carbalcoxyaminophénols, l'α-naphtol, les coupleurs possédant un groupe méthylène actif, tels que les composés β-cétoniques et les pyrazolones.

Parmi les méta-diphénols, on peut citer :
la résorcine,
la méthyl-2 résorcine,
la méthyl-5 résorcine,
le dihydroxy-2,4 phénoxyéthanol,
le monométhyléther de résorcine,
le dihydroxy-2,4 anisole.

Parmi les méta-aminophénols, on peut citer :
le méta-aminophénol,
le méthyl-2 amino-5 phénol,
le méthyl-2 N-(β-hydroxyéthyl)amino-5 phénol,
le méthyl-2 N-(β-mésylaminoéthyl)amino-5 phénol,
le diméthyl-2,6 amino-3 phénol,
l'hydroxy-6 benzomorpholine,
et leurs sels.

Parmi les métaphénylènediamines, on peut citer :
la méta-phénylènediamine,
le diamino-2,4 phénoxyéthanol,
le diméthoxy-2,4 diamino-1,3 benzène,
le triméthoxy-1,3,5 diamino-2,4 benzène,
le diamino-2,4 anisole,
l'amino-6 benzomorpholine,
le [N-(β-hydroxyéthyl)amino-2 amino-4]phénoxyéthanol,
le [N-(β-hydroxyéthyl)amino-4 amino-2]phénoxyéthanol,
l'amino-2 N-(β-hydroxyéthyl)amino-4 anisole,
le di-(β-hydroxyéthoxy)-4,5 diamino-1,3 benzène,
le β-hydroxyéthoxy-1 diamino-2,4 benzène,
et leurs sels.

Parmi les autres coupleurs utilisables dans les compositions tinctoriales de l'invention, il faut citer plus particulièrement :
le méthylènedioxy-3,4 phénol,
la méthylènedioxy-3,4 aniline,
le bromo-2 méthylènedioxy 4,5-phénol,
le chloro-2 méthylènedioxy-4,5 phénol
la méthoxy-2 méthylènedioxy-4,5 aniline.

Le poids total des précurseurs de colorants d'oxydation et des coupleurs utilisés dans les compositions tinctoriales selon l'invention est de préférence de 0,15 à 7% en poids du poids total de la composition tinctoriale.

Les compositions tinctoriales selon l'invention peuvent également renfermer des colorants directs tels que des colorants azoïques, anthraquinoniques et les dérivés nitrés de la série benzénique, qui permettent de nuancer ou d'enrichir en reflets les colorations apportées par les précurseurs de colorants d'oxydation et/ou

les coupleurs.

Le pH de la composition tinctoriale est généralement compris entre 8 et 11 et de préférence entre 9 et 11. Ce pH est ajusté à la valeur désirée à l'aide d'un agent alcalinisant tel que l'ammoniaque, les carbonates alcalins, les alcanolamines telles que la mono-, la di- ou la triéthanolamine.

Les compositions tinctoriales conformes à l'invention contiennent dans leur forme de réalisation préférée des agents tensio-actifs anioniques, cationiques, non-ioniques, amphotères ou leurs mélanges. Ces agents tensio-actifs sont présents dans les compositions conformes à l'invention dans des proportions comprises entre 0,5 et 40% en poids, et de préférence entre 2 et 30% en poids par rapport au poids total de la composition.

Ces compositions peuvent également contenir des solvants organiques pour solubiliser les composés qui ne seraient pas suffisamment solubles dans l'eau. Parmi ces solvants, on peut mentionner à titre d'exemple, les alcools en $C_1$-$C_8$, tels que l'éthanol, l'isopropanol, l'alcool benzylique et l'alcool phényléthylique; le glycérol; les glycols ou éthers de glycol comme le butoxy-2 éthanol, l'éthylèneglycol, le propylèneglycol, le monoéthyléther et le monométhyléther du diéthylèneglycol, ainsi que les produits analogues et leurs mélanges. Les solvants sont présents de préférence dans une proportion comprise entre 1 et 40% en poids, et en particulier entre 2 et 30% en poids par rapport au poids total de la composition.

Les agents épaississants que l'on peut ajouter dans les compositions conformes à l'invention sont pris notamment dans le groupe formé par l'alginate de sodium, la gomme arabique, les dérivés de la cellulose, tels que la méthylcellulose, l'hydroxyéthylcellulose, l'hydroxypropylcellulose, l'hydroxyméthylcellulose, la carboxyméthylcellulose, les polymères d'acide acrylique, la gomme de xanthane. On peut également utiliser des agents épaississants minéraux tels que la bentonite. Ces agents épaississants sont présents de préférence en des proportions comprises entre 0,1 et 5% en poids, et en particulier entre 0,5 et 3% en poids du poids total de la composition.

Les compositions peuvent contenir des agents antioxydants choisis en particulier parmi le sulfite de sodium, l'acide thioglycolique, le bisulfite de sodium, l'acide ascorbique et l'hydroquinone. Ces agents antioxydants sont présents dans la composition dans des proportions comprises entre 0,05 et 1,5% en poids du poids total de la composition.

D'autres adjuvants utilisables conformément à l'invention sont par exemple des agents de pénétration, des agents séquestrants, des tampons et des parfums.

Les compositions tinctoriales conformes à l'invention peuvent se présenter sous des formes diverses telles que sous forme de liquides, de crèmes, de gels ou sous toute autre forme appropriée, pour réaliser une teinture des fibres kératiniques et notamment des cheveux humains. Elles peuvent également être conditionnées en flacons aérosols en présence d'un agent propulseur.

Les compositions tinctoriales selon l'invention sont utilisées dans un procédé de teinture des cheveux mettant en oeuvre la révélation par un oxydant.

Conformément à ce procédé, on mélange au moment de l'emploi la composition tinctoriale décrite ci-dessus avec une solution oxydante, en une quantité suffisante pour oxyder les précurseurs de colorants d'oxydation, ensuite on applique sur les cheveux le mélange obtenu.

La solution oxydante contient en solution aqueuse des agents d'oxydation choisis dans le groupe formé par l'eau oxygénée, le peroxyde d'urée et les persels, tels que le persulfate d'ammonium. On utilise de préférence une solution d'eau oxygénée à 6% en poids (20 volumes).

Selon le procédé de teinture généralement utilisé, le mélange obtenu est appliqué sur les cheveux, à la température ambiante ou à une température ne dépassant pas 40°C, on le laisse poser pendant 10 à 40 minutes, et de préférence pendant 15 à 30 minutes, après quoi, on rince les cheveux, on les lave au shampooing, on les rince à nouveau et on les sèche.

Un autre procédé de mise en oeuvre du précurseur de colorant d'oxydation de formule (I'), conforme à l'invention, consiste à teindre les cheveux suivant un procédé en plusieurs temps, selon lequel, dans un premier temps, on applique le précurseur de colorant d'oxydation de type para au moyen d'une composition susdéfinie et dans un second temps, on applique le ou les coupleurs. L'agent oxydant est présent dans la composition appliquée dans le second temps ou bien appliquée sur les cheveux eux-mêmes dans un troisième temps, les conditions de pose, de lavage et de séchage étant identiques à celles indiquées pour le procédé en une seule étape décrit ci-dessus.

L'invention est illustrée par les exemples ci-après.

## EXEMPLES DE PREPARATION

### EXEMPLE 1

Préparation de l'amino-4[(β-hydroxyéthylthio)méthyl]-2 phénol.

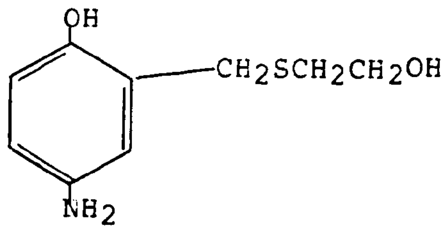

A une solution de 16 g de soude (NaOH) en pastilles dans 135 ml d'eau, on ajoute 0,085 mole (19,5 g) de nitro-4[(β-hydroxyéthylthio)méthyl]-2 phénol, puis par portions, de façon à maintenir la température entre 70°C et 75°C, 55 g d'hydrosulfite de sodium. L'agitation est maintenue 20 minutes à 75°C après la fin de l'addition. Le produit attendu est précipité, par neutralisation du milieu réactionnel par l'acide acétique, après refroidissement. Après essorage, puis lavage à l'eau et séchage, le produit obtenu est recristallisé de l'acétonitrile. Il fond à 121°C.

L'analyse du produit obtenu donne les résultats suivants :

Calculé pour $C_9H_{13}NO_2S$

| | C | H | N | O | S |
|---|---|---|---|---|---|
| Analyse | 54,24 | 6,58 | 7,03 | 16,06 | 16,09 |
| Trouvé | 54,02 | 6,62 | 7,00 | 16,17 | 15,92 |

### EXEMPLE 2

Préparation de l'amino-4[(méthylthio)méthyl]-2 phénol.

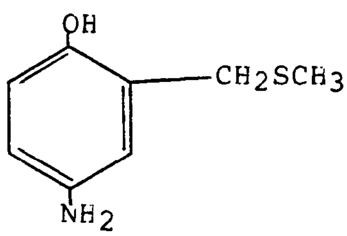

A une solution de 16 g de soude (NaOH) en pastilles dans 135 ml d'eau, on ajoute 0,085 mole (17 g) de nitro-4[(méthylthio)méthyl]-2 phénol, puis par portions de façon à maintenir la température entre 70°C et 75°C, 55 g d'hydrosulfite de sodium. L'agitation est maintenue 30 minutes à 75°C après la fin de l'addition. Le produit attendu est obtenu, par neutralisation du milieu réactionnel par l'acide acétique, après refroidissement. Après essorage, puis lavage à l'eau et séchage, il est recristallisé de l'éthanol à 96°. Il fond à 166°C.

L'analyse du produit obtenu donne les résultats suivants :

Calculé pour $C_8H_{11}NOS$

| | C | H | N | O | S |
|---|---|---|---|---|---|
| Analyse | 56,79 | 6,55 | 8,28 | 9,45 | 18,91 |
| Trouvé | 56,69 | 6,49 | 8,25 | 9,56 | 18,76 |

EXEMPLE 3

Préparation de l'hydrate d'amino-4[(β-hydroxyéthoxy)méthyl]-2 phénol.

1ère étape :

Préparation du [(β-hydroxyéthoxy)méthyl]-2 nitro-4 phénol.

On chauffe 4 heures au bain-marie bouillant 6,5 moles (93,8 g)de chlorure d'(hydroxy-2 nitro-5)benzyle dans 150 ml d'éthylèneglycol. Le produit attendu cristallise après dilution du milieu réactionnel par un litre d'eau glacée. Après essorage puis séchage à 40°C sous vide d'air, le produit obtenu est recristallisé de l'acétate d'isopropyle. Il fond à 132°C.

L'analyse du produit obtenu donne les résultats suivants :

Calculé pour $C_9H_{11}NO_5$

| | C | H | N | O |
|---|---|---|---|---|
| Analyse | 50,71 | 5,20 | 6,57 | 37,52 |
| Trouvé | 50,77 | 5,21 | 6,48 | 37,77 |

2ème étape :

Préparation de l'hydrate d'amino-4[(β-hydroxyéthoxy)méthyl]-2 phénol.

On chauffe sous agitation au reflux 25 g de zinc en poudre fine et 0,5 g de chlorure d'ammonium dans 10 ml d'eau et 50 ml d'éthanol à 96°. On ajoute par portions 0,05 mole (10,6 g) de [(β-hydroxyéthoxy)méthyl]-2 nitro-4 phénol préparé à l'étape précédente, le chauffage est prolongé 10 minutes après la fin de l'addition. On filtre bouillant le zinc. Le filtrat est évaporé à sec, dilué à l'acétate d'éthyle puis purifié par passage sur une colonne de silice (éluant : acétate d'éthyle 40/cyclohexane 60). Le produit obtenu est recristallisé de l'acétonitrile. Il fond à 57°C.

L'analyse du produit donne les résultats suivants :

Calculé pour $C_9H_{15}NO_4$

| | C | H | N |
|---|---|---|---|
| Calculé | 53,72 | 7,51 | 6,96 |
| Trouvé | 53,84 | 7,23 | 7,01 |

EXEMPLE 4

Préparation de l'amino-4[(β,γ-dihydroxypropylthio)méthyl]-2 phénol.

1ère étape :

Préparation du [(β,γ-dihydroxypropylthio)méthyl]-2 nitro-4 phénol.

On chauffe à 45°C 13,2 g de potasse en pastilles (85%) dans 27 g de thioglycérol. On ajoute 10 ml de N-méthylpyrrolidone, puis par portions en 30 minutes, 0,1 mole (23,2 g) de bromure d'hydroxy-2 nitro-5 benzyle. La température de 67-70°C obtenue est maintenue 30 minutes après la fin de l'addition. Le mélange réactionnel est dilué par 300 g d'eau glacée. Par neutralisation à l'acide acétique, le produit attendu précipite. Après essorage puis séchage à 45°C sous vide, le produit obtenu est recristallisé de l'acétate d'isopropyle. Il fond à 130°C.

L'analyse du produit obtenu donne les résultats suivants :

Calculé pour $C_{10}H_{13}NO_5S$

| | C | H | N | O | S |
|---|---|---|---|---|---|
| Analyse | 46,32 | 5,05 | 5,40 | 30,85 | 12,37 |
| Trouvé | 46,29 | 5,07 | 5,44 | 30,84 | 12,25 |

2ème étape :

Préparation de l'amino-4[(β,γ-dihydroxypropylthio)méthyl]-2 phénol.

A une solution de 8 g d'hydroxyde de sodium en pastille dans 70 ml d'eau, on ajoute 0,05 mole (13 g) de [(β,γ -dihydroxypropylthio)méthyl]-2 nitro-4 phénol, puis par portions, de façon à maintenir la température entre 70 et 75°C, 28 g d'hydrosulfite de sodium. L'agitation est maintenue 1 heure à 75°C après la fin de l'addition. Le produit attendu est extrait à l'acétate d'éthyle après neutralisation du milieu réactionnel par l'acide acétique. Il est purifié par chromatographie sur colonne de silice (éluant:acétate d'éthyle). Après recristallisation de l'acétonitrile, il fond à 90°C.

L'analyse du produit obtenu donne les résultats suivants :

Calculé pour $C_{10}H_{15}NO_3S$

| | C | H | N | O | S |
|---|---|---|---|---|---|
| Analyse | 52,38 | 6,59 | 6,11 | 20,93 | 13,98 |
| Trouvé | 52,36 | 6,57 | 6,01 | 21,07 | 13,90 |

EXEMPLE 5

Préparation de l'amino-4[(trifluoro-2',2',2'éthoxy)méthyl]-2 phénol.

1ère étape :

Préparation du nitro-4[(trifluoro-2',2',2'éthoxy)méthyl]-2 phénol.

On mélange 13,2 g d'hydroxyde de potassium en pastille (85%) et 0,25 mole (25 g) de trifluoro-2,2,2 éthanol dans 10 ml de N-méthylpyrrolidone à 65°C. On ajoute par portions en 30 minutes 0,1 mole (23,2 g) de bromure d'hydroxy-2 nitro-5 benzyle. La température est maintenue 1 heure à 85°C après la fin de l'addition.

Le mélange réactionnel est dilué par 300 g d'eau glacée. Par neutralisation par l'acide acétique, le produit attendu décante. Il est purifié sur colonne de silice (éluant : acétate d'éthyle/cyclohexane 20/80). Le produit obtenu cristallise lentement. Il fond à 60°C.

L'analyse du produit obtenu donne les résultats suivants :

Calculé pour $C_9H_8NO_4F_3$

| | C | H | N | F |
|---|---|---|---|---|
| Analyse | 43,04 | 3,21 | 5,58 | 22,69 |
| Trouvé | 43,20 | 3,13 | 5,50 | 22,65 |

2ème étape :

Préparation de l'amino-4[(trifluoro-2',2',2')éthoxy méthyl]-2 phénol.

On chauffe 30 minutes au reflux 10 g de zinc en poudre et 0,2 g de chlorure d'ammonium dans 20 ml d'éthanol et 4 ml d'eau. On ajoute par portions 5 g de nitro-4[(trifluoro-2',2',2'éthoxy)méthyl]-2 phénol (0,02 mole). Le chauffage est maintenu 30 minutes après la fin de l'addition. On élimine le zinc par filtration bouillante. Le produit attendu est obtenu par évaporation du filtrat sous vide. Recristallisé d'un mélange cyclohexane-acétate d'éthyle, il fond à 113°C.

L'analyse du produit obtenu donne les résultats suivants :

Calculé pour $C_9H_{10}NO_2F_3$

| | C | H | N | F |
|---|---|---|---|---|
| Analyse | 48,87 | 4,56 | 6,33 | 25,77 |
| Trouvé | 48,83 | 4,60 | 6,23 | 25,68 |

EXEMPLES D'APPLICATION

EXEMPLE A1

On prépare le mélange tinctorial suivant :

| | | |
|---|---|---|
| - Amino-4 méthoxyméthyl-2 phénol | | 0,383 g |
| - [(ß-hydroxyéthyl)amino]-4 hydroxy-2 toluène | | 0,417 g |
| - Alcool oléique polyglycérolé à 2 moles de glycérol | | 4,5 g |
| - Alcool oléique polyglycérolé à 4 moles de glycérol | | 4,5 g |
| - Oléylamine oxyéthylénée à 12 moles d'oxyde d'éthylène, vendue sous la dénomination ETHOMEEN O 12 par ARMOON HESS CHEMICAL Ltd | | 4,5 g |
| - Diéthanolamide de coprah vendu sous la dénomination COMPERLAN KD par HENKEL | | 9,0 g |
| - Propylèneglycol | | 4,0 g |
| - 2-butoxyéthanol | | 8,0 g |
| - Ethanol à 96° | | 6,0 g |
| - Sel pentasodique de l'acide diéthylène triamine pentacétique, vendu sous la dénomination MASQUOL DTPA par PROTEX | | 2,0 g |
| - Hydroquinone | | 0,15 g |
| - Solution de bisulfite de sodium à 35° Bé | | 1,3 g |
| - Ammoniaque à 22° Bé | | 10,0 g |
| - Eau | qsp | 100,0 g |
| - pH = 10 | | |

Au moment de l'emploi, on ajoute 100 g d'eau oxygénée à 20 volumes (6% en poids). Le mélange, appliqué 20 minutes à 35°C sur cheveux décolorés leur confère, après shampooing et rinçage, une coloration orangée.

EXEMPLE A2

On prépare le mélange tinctorial suivant :

| | | | |
|---|---|---|---|
| - Amino-4 [(méthylthio)méthyl]-2 phénol | | 0,423 | g |
| - [(ß-hydroxyéthyl)amino]-4 hydroxy-2 toluène | | 0,417 | g |
| - Alcool oléique polyglycérolé à 2 moles de glycérol | | 4,5 | g |
| - Alcool oléique polyglycérolé à 4 moles de glycérol | | 4,5 | g |
| - Oléylamine oxyéthylénée à 12 moles d'oxyde d'éthylène, vendue sous la dénomination ETHOMEEN O 12 par ARMOON HESS CHEMICAL Ltd | | 4,5 | g |
| - Diéthanolamide de coprah vendu sous la dénomination COMPERLAN KD par HENKEL | | 9,0 | g |
| - Propylèneglycol | | 4,0 | g |
| - 2-butoxyéthanol | | 8,0 | g |
| - Ethanol à 96° | | 6,0 | g |
| - Sel pentasodique de l'acide diéthylène triamine pentacétique, vendu sous la dénomination MASQUOL DTPA par PROTEX | | 2,0 | g |
| - Hydroquinone | | 0,15 | g |
| - Solution de bisulfite de sodium à 35° Bé | | 1,3 | g |
| - Ammoniaque à 22° Bé | | 10,0 | g |
| - Eau | qsp | 100,0 | g |
| - pH = 10 | | | |

Au moment de l'emploi, on ajoute 100 g d'eau oxygénée à 20 volumes (6% en poids). Le mélange, appliqué 20 minutes à 35°C sur cheveux décolorés leur confère, après shampooing et rinçage, une coloration brun orangé clair.

EXEMPLE A3

On prépare le mélange tinctorial suivant :

| | | |
|---|---|---|
| - Amino-4 méthoxyméthyl-2 phénol | | 0,383 g |
| - Dichlorhydrate de (diamino-2,4 phénoxy) éthanol | | 0,602 g |
| - Alcool oléique polyglycérolé à 2 moles de glycérol | | 4,5 g |
| - Alcool oléique polyglycérolé à 4 moles de glycérol | | 4,5 g |
| - Oléylamine oxyéthylénée à 12 moles d'oxyde d'éthylène, vendue sous la dénomination ETHOMEEN O 12 par ARMOON HESS CHEMICAL Ltd | | 4,5 g |
| - Diéthanolamide de coprah vendu sous la dénomination COMPERLAN KD par HENKEL | | 9,0 g |
| - Propylèneglycol | | 4,0 g |
| - 2-butoxyéthanol | | 8,0 g |
| - Ethanol à 96° | | 6,0 g |
| - Sel pentasodique de l'acide diéthylène triamine pentacétique, vendu sous la dénomination MASQUOL DTPA par PROTEX | | 2,0 g |
| - Hydroquinone | | 0,15 g |
| - Solution de bisulfite de sodium à 35° Bé | | 1,3 g |
| - Ammoniaque à 22° Bé | | 10,0 g |
| - Eau | qsp | 100,0 g |
| - pH = 10 | | |

Au moment de l'emploi, on ajoute 100 g d'eau oxygénée à 20 volumes (6% en poids). Le mélange, appliqué 20 minutes à 35°C sur cheveux naturellement blancs à 90% leur confère, après shampooing et rinçage, une coloration brun rouge.

EXEMPLE A4

On prépare le mélange tinctorial suivant :

| | | |
|---|---|---|
| - Amino-4[(méthylthio)méthyl]-2 phénol | | 0,423 g |
| - Dichlorhydrate de (diamino-2,4 phénoxy) éthanol | | 0,602 g |
| - Alcool oléique polyglycérolé à 2 moles de glycérol | | 4,5 g |
| - Alcool oléique polyglycérolé à 4 moles de glycérol | | 4,5 g |
| - Oléylamine oxyéthylénée à 12 moles d'oxyde d'éthylène, vendue sous la dénomination ETHOMEEN O 12 par ARMOON HESS CHEMICAL Ltd | | 4,5 g |
| - Diéthanolamide de coprah vendu sous la dénomination COMPERLAN KD par HENKEL | | 9,0 g |
| - Propylèneglycol | | 4,0 g |
| - 2-butoxyéthanol | | 8,0 g |
| - Ethanol à 96° | | 6,0 g |
| - Sel pentasodique de l'acide diéthylène triamine pentacétique, vendu sous la dénomination MASQUOL DTPA par PROTEX | | 2,0 g |
| - Hydroquinone | | 0,15 g |
| - Solution de bisulfite de sodium à 35° Bé | | 1,3 g |
| - Ammoniaque à 22° Bé | | 10,0 g |
| - Eau | qsp | 100,0 g |
| - pH = 10 | | |

Au moment de l'emploi, on ajoute 100 g d'eau oxygénée à 20 volumes (6% en poids). Le mélange, appliqué 20 minutes à 35°C sur cheveux naturellement blancs à 90% leur confère, après shampooing et rinçage, une coloration rouge-gris.

EXEMPLE A5

On prépare le mélange tinctorial suivant :

| | | |
|---|---|---|
| - Amino-4 [(ß-hydroxyéthylthio)méthyl]-2 phénol | | 0,99 g |
| - Amino-4 hydroxy-2 toluène | | 0,62 g |
| - Alcool oléique polyglycérolé à 2 moles de glycérol | | 4,5 g |
| - Alcool oléique polyglycérolé à 4 moles de glycérol | | 4,5 g |
| - Oléylamine oxyéthylénée à 12 moles d'oxyde d'éthylène, vendue sous la dénomination ETHOMEEN O 12 par ARMOON HESS CHEMICAL Ltd | | 4,5 g |
| - Diéthanolamide de coprah vendu sous la dénomination COMPERLAN KD par HENKEL | | 9,0 g |
| - Propylèneglycol | | 4,0 g |
| - 2-butoxyéthanol | | 8,0 g |
| - Ethanol à 96° | | 6,0 g |
| - Sel pentasodique de l'acide diéthylène triamine pentacétique, vendu sous la dénomination MASQUOL DTPA par PROTEX | | 2,0 g |
| - Hydroquinone | | 0,15 g |
| - Solution de bisulfite de sodium à 35° Bé | | 1,3 g |
| - Ammoniaque à 22° Bé | | 10,0 g |
| - Eau | qsp | 100,0 g |
| - pH =10 | | |

Au moment de l'emploi, on ajoute 100 g d'eau oxygénée à 20 volumes (6% en poids). Le mélange, appliqué 20 minutes à 35°C sur cheveux naturellement blancs à 90% leur confère, après shampooing et rinçage, une coloration orange doré.

EXEMPLE A6

On prépare le mélange tinctorial suivant :

| | | |
|---|---|---|
| - Amino-4 méthoxyméthyl-2 phénol | | 0,215 g |
| - paraphénylènediamine | | 0,199 g |
| - Résorcine | | 0,197 g |
| - Métaaminophénol | | 0,272 g |
| - 2-butoxyéthanol | | 10,0 g |
| - Nonylphénol oxyéthylèné à 4 moles d'oxyde d'éthylène, vendu sous la dénomination CEMULSOL NP 4 par RHONE POULENC | | 12,0 g |
| - Nonylphénol oxyéthylèné à 9 moles d'oxyde d'éthylène, vendu sous la dénomination CEMULSOL NP 9 par RHONE POULENC | | 15,0 g |
| - Alcool oléique polyglycérolé à 2 moles de glycérol | | 1,5 g |
| - Alcool oléique polyglycérolé à 4 moles de glycérol | | 1,5 g |
| - Propylèneglycol | | 6,0 g |
| - Acide éthylènediamine tétracétique vendu sous la dénomination TRILON B | | 0,12 g |
| - Ammoniaque à 22° Bé | | 11,0 g |
| - Acide thioglycolique | | 0,6 g |
| - Eau | qsp | 100,0 g |
| - pH = 10,3 | | |

Au moment de l'emploi, on ajoute 100 g d'eau oxygénée à 20 volumes (6% en poids). Le mélange, appliqué 20 minutes à 35°C sur cheveux naturellement blancs à 90% leur confère, après shampooing et rinçage, une coloration châtain.

EXEMPLE A7

On prépare le mélange tinctorial suivant :

| | | |
|---|---|---|
| - Amino-4/(méthylthio)méthyl7-2 phénol | | 0,177 g |
| - Dichlorhydrate de (ß-méthoxyéthyl) amino-4 aniline | | 1,177 g |
| - Chlorhydrate de méthylamino-4 phénol | | 0,407 g |
| - Métaaminophénol | | 0,195 g |
| - Dichlorhydrate de diamino-1,3 diméthoxy-2,4 benzène | | 0,085 g |
| - Résorcine | | 0,385 g |
| - Hydroxy-6 benzomorpholine | | 0,074 g |
| - 2-butoxyéthanol | | 10,0 g |
| - Alcool cétylstéarylique vendu sous la dénomination ALFOL C 16/18 par CONDEA | | 8,0 g |
| - Cétylstéarylsulfate de sodium vendu sous la dénomination CIRE DE LANETTE E par HENKEL | | 0,5 g |
| - Huile de ricin éthoxylée vendue sous la dénomination CEMULSOL B par RHONE POULENC | | 1,0 g |
| - Diéthanolamide oléique | | 1,5 g |
| - Sel pentasodique de l'acide diéthylène triamine pentacétique vendu sous la dénomination MASQUOL DTPA par PROTEX | | 2,5 g |
| - Ammoniaque à 22° Bé | | 11,0 g |
| - Eau | qsp | 100,0 g |
| - pH = 10,2 | | |

Au moment de l'emploi, on ajoute 100 g d'eau oxygénée à 20 volumes (6% en poids). Le mélange, appliqué 20 minutes à 35°C sur cheveux décolorés leur confère, après shampooing et rinçage, une coloration bronze cendré.

EXEMPLE A8

On prépare le mélange tinctorial suivant :

| | | |
|---|---|---|
| - Amino-4 éthoxyméthyl-2 phénol | | 0,84 g |
| - Amino-4 hydroxy-2 toluène | | 0,62 g |
| - 2-butoxyéthanol | | 10,0 g |
| - Alcool cétylstéarylique vendu sous la dénomination ALFOL C 16/18 par la Société CONDEA | | 8,0 g |
| - Cétylstéarylsulfate de sodium vendu sous la dénomination CIRE DE LANETTE E par la Société HENKEL | | 0,5 g |
| - Huile de ricin éthoxylée vendu sous la dénomination CEMULSOL B par la Société RHONE POULENC | | 1,0 g |
| - Diéthanolamide oléique | | 1,5 g |
| - Sel pentasodique de l'acide diéthylène triamine pentacétique vendu sous la dénomination MASQUOL DTPA par la Société PROTEX | | 2,5 g |
| - Ammoniaque à 22°Bé | | 11,0 g |
| - Eau | qsp | 100,0 g |
| - pH = 10,8 | | |

Au moment de l'emploi, on ajoute 100 g d'eau oxygénée à 20 volumes. Le mélange, appliqué 20 minutes à 30°C sur cheveux naturellement blancs à 90% leur confère, après shampooing et rinçage, une coloration orangé.

EXEMPLE A9

On prépare le mélange tinctorial suivant :

| | | |
|---|---|---|
| - Amino-4 [(ß-hydroxyéthoxy)méthyl]-2 phénol | | 0,451 g |
| - [(ß-hydroxyéthyl)amino]-4 hydroxy-2 toluène | | 0,417 g |
| - Nonylphénol oxyéthyléné à 4 moles d'oxyde d'éthylène vendu sous la dénomination CEMULSOL NP 4 par la Société RHONE POULENC | | 12,0 g |
| - Nonylphénol oxyéthyléné à 9 moles d'oxyde d'éthylène vendu sous la dénomination CEMULSOL NP 9 par la Société RHONE POULENC | | 15,0 g |
| - Alcool oléique polyglycérolé à 2 moles de glycérol | | 1,5 g |
| - Alcool oléique polyglycérolé à 4 moles de glycérol | | 1,5 g |
| - Propylèneglycol | | 6,0 g |
| - Acide éthylènediamine tétracétique vendu sous la dénomination TRILON B | | 0,12 g |
| - Ammoniaque à 22°Bé | | 11,0 g |
| - Eau | qsp | 100,0 g |
| - pH = 10,15 | | |

Au moment de l'emploi, on ajoute 90 g d'eau oxygénée à 20 volumes. Le mélange, appliqué 25 minutes à 30°C sur cheveux décolorés leur confère, après shampooing et rinçage, une coloration orangé vif.

EXEMPLE A10

On prépare le mélange tinctorial suivant :

| | | |
|---|---|---|
| - Amino-4 [(ß, γ-dihydroxypropylthio) méthyl]-2 phénol | | 0,286 g |
| - Dichlorhydrate de (diamino-2,4 phénoxy)éthanol | | 0,301 g |
| - Alcool cétylstéarylique vendu sous la dénomination ALFOL C 16/18 par la Société CONDEA | | 19,0 g |
| - Octyl-2 dodécanol vendu sous la dénomination EUTANOL G par la Société HENKEL | | 4,5 g |
| - Alcool cétylstéarylique à 15 moles d'oxyde d'éthylène vendu sous la dénomination MERGITAL C.S. par la Société HENKEL | | 2,5 g |
| - Laurylsulfate d'ammonium | | 10,0 g |
| - Polymère cationique présentant le motif récurrent suivant : 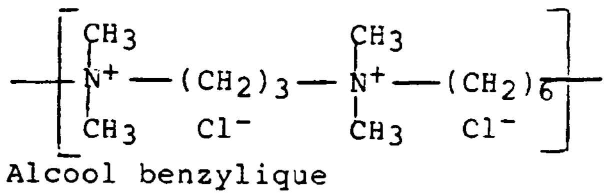 | | 4,0 g |
| - Alcool benzylique | | 2,0 g |
| - Ammoniaque à 22°Bé | | 11 ml |
| - Acide éthylènediamine tétracétique vendu sous la dénomination TRILON B | | 1,0 g |
| - Solution de bisulfite de sodium à 35°Bé | | 1,2 g |
| - Eau | qsp | 100,0 g |
| - pH = 10 | | |

Au moment de l'emploi, on ajoute 100 g d'eau oxygénée à 20 volumes. Le mélange, appliqué 20 minutes à 30°C sur cheveux décolorés leur confère, après shampooing et rinçage, une coloration prune gris.

EXEMPLE A11

On prépare le mélange tinctorial suivant :

| | | | |
|---|---|---|---|
| - Hydrate d'amino-4 /(ß-hydroxyéthoxy) méthyl/-2 phénol | | 1,3 | g |
| - p-phénylènediamine | | 0,2 | g |
| - Dichlorhydrate de (diamino-2,4 phénoxy) éthanol | | 0,05 | g |
| - Trifluoroéthoxy-2 méthylènedioxy-4,5 aniline | | 0,05 | g |
| - Hydroxyéthylcellulose vendue sous la dénomination CELLOSIZE WP 03 par la Société UNION CARBIDE | | 2,0 | g |
| - Laurylsulfate d'ammonium | | 5,0 | g |
| - 2-butoxyéthanol | | 15,0 | g |
| - Ethanol à 96° | | 6,0 | g |
| - Ammoniaque à 22°Bé | | 10,0 | g |
| - Solution de bisulfite de sodium à 35°Bé | | 1,5 | g |
| - Hydroquinone | | 0,15 | g |
| - Eau | qsp | 100,0 | g |
| - pH = 10,2 | | | |

Au moment de l'emploi, on ajoute 100 g d'eau oxygénée à 20 volumes. Le mélange, appliqué 25 minutes à 30°C sur cheveux naturellement blancs à 90% leur confère, après shampooing et rinçage, une coloration gris beige cendré.

EXEMPLE A12

On prépare le mélange tinctorial suivant :

| | |
|---|---|
| - Amino-4 éthoxyméthyl-2 phénol | 1,04 g |
| - [(ß-hydroxyéthyl)amino]-4 hydroxy-2 toluène | 0,5 g |
| - Méta-aminophénol | 0,227 g |
| - Bromo-2 méthylènedioxy-4,5 phénol | 0,057 g |
| - Chlorhydrate d'acétoxy-4 hydroxy-2 aniline | 0,503 g |
| - 2-butoxyéthanol | 10,0 g |
| - Nonylphénol oxyéthyléné à 4 moles d'oxyde d'éthylène vendu sous la dénomination CEMULSOL NP 4 par la Société RHONE POULENC | 12,0 g |
| - Nonylphénol oxyéthyléné à 9 moles d'oxyde d'éthylène vendu sous la dénomination CEMULSOL NP 9 par la Société RHONE POULENC | 15,0 g |
| - Alcool oléique polyglycérolé à 2 moles de glycérol | 1,5 g |
| - Alcool oléique polyglycérolé à 4 moles de glycérol | 1,5 g |
| - Propylèneglycol | 6,0 g |
| - Acide éthylènediamine tétracétique vendu sous la dénomination TRILON B | 0,12 g |
| - Ammoniaque à 22°Bé | 11,0 g |
| - Acide thioglycolique | 0,6 g |
| - Eau qsp | 100,0 g |
| - pH = 9,7 | |

Au moment de l'emploi, on ajoute 80 g d'eau oxygénée à 20 volumes. Le mélange, appliqué 25 minutes à 30°C sur cheveux naturellement blancs à 90% leur confère, après shampooing et rinçage, une coloration vieux rose.

EXEMPLE A13

On prépare le mélange tinctorial suivant :

| | |
|---|---|
| - Amino-4 [(trifluoro-2',2',2'éthoxy) méthyl]-2 phénol | 0,55 g |
| - Diamino-2,4 phénoxyéthanol | 0,60 g |
| - Alcool cétylstéarylique vendu sous la dénomination ALFOL C 16/18 par la Société CONDEA | 19,0 g |
| - 2-octyldodécanol vendu sous la dénomination EUTANOL G par la Société HENKEL | 4,5 g |
| - Alcool cétylstéarylique à 15 moles d'oxyde d'éthylène vendu sous la dénomination MERGITAL C.S. par la Société HENKEL | 2,5 g |
| - Laurylsulfate d'ammonium | 10,0 g |
| - Polymère cationique présentant le motif récurrent suivant : 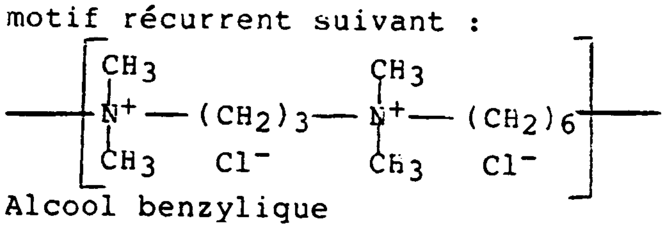 | 4,0 g |
| - Alcool benzylique | 2,0 g |
| - Ammoniaque à 22°Bé | 11 ml |
| - Acide éthylènediamine tétracétique vendu sous la dénomination TRILON B | 1,0 g |
| - Solution de bisulfite de sodium à 35°Bé | 1,2 g |
| - Eau qsp | 100,0 g |
| - pH = 9,4 | |

Au moment de l'emploi, on ajoute 90 g d'eau oxygénée à 20 volumes. Le mélange, appliqué 20 minutes à 35°C sur cheveux décolorés leur confère, après shampooing et rinçage, une coloration violine.

EXEMPLE A14

On prépare le mélange tinctorial suivant :

| | | | |
|---|---|---|---|
| - Amino-4 /(trifluoro-2',2',2'éthoxy) méthyl/-2 phénol | | 0,66 | g |
| - Méthyl-2 amino-5 phénol | | 0,369 | g |
| - Hydroxyéthyl cellulose vendue sous la dénomination CELLOSIZE WP 03 par la Société UNION CARBIDE | | 2,0 | g |
| - Laurylsulfate d'ammonium | | 5,0 | g |
| - 2-butoxyéthanol | | 15,0 | g |
| - Ethanol à 96° | | 6,0 | g |
| - Ammoniaque à 22°Bé | | 10,0 | g |
| - Solution de bisulfite de sodium à 35°Bé | | 1,5 | g |
| - Hydroquinone | | 0,15 | g |
| - Eau | qsp | 100,0 | g |
| - pH = 10,3 | | | |

Au moment de l'emploi, on ajoute 100 g d'eau oxygénée à 20 volumes. Le mélange, appliqué 25 minutes à 35°C sur cheveux décolorés leur confère, après shampooing et rinçage, une coloration orange.

**Revendications**

**Revendications pour les Etats contractants suivants : AT, BE, CH, DE, FR, GB, GR, IT, LI, NL, SE**

**1.** Para-aminophénols 2-substitués de formule (I) :

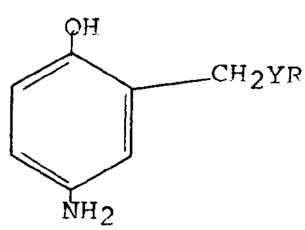

(I)

dans laquelle Y représente un atome d'oxygène ou de soufre et R représente un radical alkyle en $C_1$-$C_6$, un radical hydroxyalkyle en $C_1$-$C_6$, polyhydroxyalkyle en $C_2$-$C_6$ ou halogénoalkyle en $C_1$-$C_6$, avec la condition que lorsque Y désigne oxygène, R n'est pas méthyle ou éthyle et lorsque Y désigne soufre, R n'est pas éthyle; et leurs sels, à l'exclusion du 4-amino 2-[(2',2',2'-trifluoroéthoxy)méthyl]phénol.

**2.** Para-aminophénols 2-substitués selon la revendication 1, caractérisés par le fait que Y désigne un atome de soufre et R désigne un radical choisi parmi les radicaux hydroxy-2 éthyle, méthyle et β,γ-dihydroxypropyle.

**3.** Para-aminophénols 2-substitués selon la revendication 1, caractérisés par le fait que Y désigne un atome d'oxygène et R désigne le radical β-hydroxyéthyle.

**4.** Para-aminophénols 2-substitués selon les revendications 1 à 3, caractérisés par le fait qu'ils se présentent sous la forme de chlorhydrate ou de sulfate.

**5.** Composition tinctoriale pour la teinture des fibres kératiniques et en particulier des cheveux humains, caractérisée par le fait qu'elle comprend, dans un véhicule aqueux, un ou plusieurs composés de formule (I') :

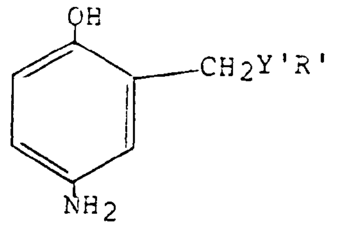

(I')

dans laquelle Y' représente un atome d'oxygène ou de soufre, R' représente un radical alkyle en $C_1$-$C_6$, hydroxyalkyle en $C_1$-$C_6$, polyhydroxyalkyle en $C_2$-$C_6$ or halogénoalkyle en $C_1$-$C_6$ ou un ou plusieurs sels d'un composé de formule (I').

6. Composition selon la revendication 5, caractérisée par le fait que le composé de formule (I') est choisi dans le groupe formé par l'amino-4-[(méthylthio)méthyl]-2 phénol, l'amino-4[(β-hydroxyéthylthio) méthyl]-2 phénol, l'amino-4 méthoxyméthyl-2 phénol; l'amino-4 éthoxyméthyl-2 phénol, l'amino-4 [(β-hydroxy éthoxy)méthyl]-2 phénol, l'amino-4 [(β,γ-dihydroxypropylthio)méthyl]-2 phénol et l'amino-4 [(trifluoro-2', 2',2'éthoxy)méthyl]-2 phénol.

7. Composition selon les revendications 5 ou 6, caractérisée par le fait qu'elle contient d'autres précurseurs de colorants par oxydation choisis parmi les para-phénylènediamines, les para-aminophénols différents de ceux de formule (I'), les ortho-phénylènediamines et les ortho-aminophénols.

8. Composition selon les revendications 5 à 7, caractérisée par le fait qu'elle comprend également un coupleur.

9. Composition selon la revendication 8, caractérisée par le fait que le coupleur est choisi dans le groupe formé par les métha-diphénols, les méta-amino-phénols, les méta-phénylènediamines, les méta-acylamino-phénols, les méta-uréidophénols, les méta-carbalcoxyaminophénols, l'alpha-naphtol, les coupleurs possédant un groupe méthylène actif tels que les composés β-cétoniques et les pyrazolones, le méthylènedioxy-3,4 phénol, la méthylènedioxy-3,4 aniline, le bromo-2 méthylènedioxy-4,5 phénol, le chloro-2 méthylènedioxy-4,5 phénol et la méthoxy-2 méthylènedioxy-4,5 aniline.

10. Composition selon les revendications 5 à 9, caractérisée par le fait que la composition comprend également un ou plusieurs colorants directs.

11. Composition selon la revendication 10, caractérisée par le fait que les colorants directs sont choisis dans le groupe formé par les colorants azoïques, les colorants anthraquinoniques et les dérivés nitrés de la série benzénique.

12. Composition selon les revendications 5 à 11, caractérisée par le fait qu'elle renferme de 0,02 à 6% en poids de composé de formule (I') par rapport au poids total de la composition.

13. Composition selon les revendications 7 à 12, caractérisée par le fait que le poids total des précurseurs de colorants d'oxydation et des coupleurs est de 0,15 à 7% en poids du poids total de la composition tinctoriale.

14. Composition selon les revendications 5 à 13, caractérisée par le fait qu'elle comprend également des adjuvants choisis parmi les agents tensio-actifs anioniques, cationiques, non-ioniques, amphotères et leurs mélanges, les solvants organiques, les agents épaississants, les agents antioxydants, les agents de pénétration, les agents séquestrants, les tampons, les parfums, les agents alcalinisants.

15. Composition selon les revendications 5 à 14, caractérisée par le fait que son pH est de 8 à 11.

16. Procédé de teinture des cheveux humains, caractérisé par le fait qu'on applique sur les cheveux pendant 10 à 40 minutes et à une température comprise entre la température ambiante et 40°C, une quantité suffisante, pour teindre les cheveux, d'une composition selon l'une quelconque des revendications 5 à 15, mélangée au moment de l'emploi avec une solution oxydante, en quantité suffisante pour oxyder les précurseurs de colorants d'oxydation, après quoi on rince les cheveux, on les lave au shampoing, on les rince à nouveau et on les sèche.

17. Procédé de teinture des cheveux en plusieurs étapes, caractérisé par le fait que :
(i) dans une première étape, on applique sur les cheveux une quantité suffisante pour les teindre, d'une composition selon l'une quelconque des revendications 5 à 7 et 10 à 15, contenant un ou plusieurs composés de formule (I'), éventuellement d'autres précurseurs de colorants d'oxydation; et
(ii) dans une deuxième étape, on applique sur les cheveux une solution contenant un ou plusieurs coupleurs, l'agent oxydant étant présent dans la composition appliquée dans la deuxième étape ou bien appliqué sur les cheveux eux-mêmes dans une troisième étape, les différentes compositions étant maintenues au contact des cheveux pendant 10 à 40 minutes à une température comprise entre la température ambiante et 40°C, après quoi on rince les cheveux, on les lave au shampooing, on les rince à nouveau et on les sèche.

**Revendications pour l'Etat contractant suivant : ES**

1. Procédé de préparation des para-amino-phénols 2-substitués de formule (I) :

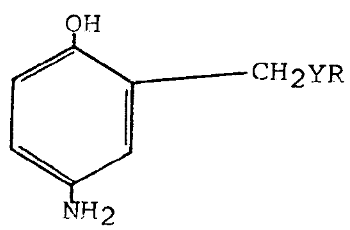
(I)

dans laquelle Y représente un atome d'oxygène ou de soufre et R représente un radical alkyle en $C_1$-$C_6$, un radical hydroxyalkyle en $C_1$-$C_6$, polyhydroxyalkyle en $C_2$-$C_6$ ou halogénoalkyle en $C_1$-$C_6$, avec la condition que lorsque Y désigne oxygène, R n'est pas méthyle ou éthyle et lorsque Y désigne soufre, R n'est pas éthyle; et leurs sels, à l'exclusion du 4-amino 2-[(2',2',2'-trifluoroéthoxy)méthyl]phénol, selon le schéma réactionnel ci-dessous :

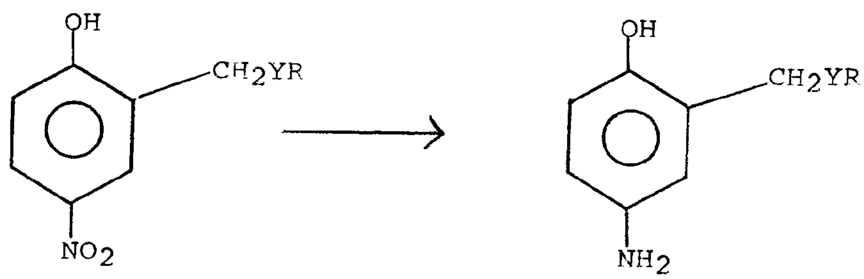
(II) (I)

par réduction du composé de formule (II) où Y et R ont les significations ci-dessus indiquées.

2. Procédé selon la revendication 1, caractérisé par le fait que la réduction est réalisée par l'hydrosulfite de sodium en milieu alcalin à une température inférieure à 80°C, ou bien par réduction catalytique en milieu hydroalcoolique, sous pression d'hydrogène, en présence d'un catalyseur tel que le Palladium sur charbon ou le Nickel.

3. Procédé de préparation selon la revendication 1 ou 2, caractérisé par le fait que dans la formule (I) Y désigne un atome de soufre et R désigne un radical choisi parmi les radicaux hydroxy-2 éthyle, méthyle et β,γ-dihydroxypropyle.

4. Procédé de préparation selon les revendications 1 ou 2, caractérisé par le fait que dans la formule (I) Y désigne un atome d'oxygène et R désigne le radical β-hydroxyéthyle.

5. Procédé de préraration selon les revendications 1 à 4, caractérisé par le fait que les para-aminophénols 2-substitués de formule (I) sont transformés en un sel acide et de préférence en chlorhydrate ou sulfate.

6. Composition tinctoriale pour la teinture des fibres kératiniques et en particulier des cheveux humains, caractérisée par le fait qu'elle comprend, dans un véhicule aqueux, un ou plusieurs composés de formule (I′) :

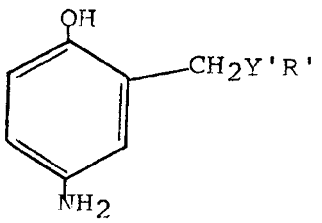

(I')

dans laquelle Y′ représente un atome d'oxygène ou de soufre, R′ représente un radical alkyle en $C_1$-$C_6$, hydroxyalkyle en $C_1$-$C_6$, polyhydroxyalkyle en $C_2$-$C_6$ ou halogénoalkyle en $C_1$-$C_6$ ou un ou plusieurs sels d'un composé de formule (I′) obtenus par un procédé selon les revendications 1 à 5.

7. Composition selon la revendication 6, caractérisée par le fait que le composé de formule (I′) est choisi dans le groupe formé par l'amino-4[(méthylthio)méthyl]-2 phénol, l'amino-4[(β-hydroxyéthylthio) méthyl]-2 phénol, l'amino-4 méthoxyméthyl-2 phénol; l'amino-4 éthoxyméthyl-2 phénol, l'amino-4 [(β-hydroxy éthoxy)méthyl]-2 phénol, l'amino-4 [(β-, γ-dihydroxypropylthio)méthyl]-2 phénol et l'amino-4 [(trifluoro-2′, 2′,2′éthoxy)méthyl]-2 phénol.

8. Composition selon les revendications 6 ou 7, caractérisée par le fait qu'elle contient d'autres précurseurs de colorants par oxydation choisis parmi les para-phénylènediamines, les para-aminophénols différents de ceux de formule (I′), les ortho-phénylènediamines et les ortho-aminophénols.

9. Composition selon les revendications 6 à 8, caractérisée par le fait qu'elle comprend également un coupleur.

10. Composition selon la revendication 9, caractérisée par le fait que le coupleur est choisi dans le groupe formé par les méta-diphénols, les méta-amino-phénols, les méta-phénylènediamines, les méta-acylamino-phénols, les méta-uréidophénols, les méta-carbalcoxyaminophénols, l'alpha-naphtol, les coupleurs possédant un groupe méthylène actif tels que les composés β-cétoniques et les pyrazolones, le méthylènedioxy-3,4 phénol, la méthylènedioxy-3,4 aniline, le bromo-2 méthylènedioxy-4,5 phénol, le chloro-2 méthylènedioxy-4,5 phénol et la méthoxy-2 méthylènedioxy-4,5 aniline.

11. Composition selon les revendications 6 à 10, caractérisée par le fait que la composition comprend également un ou plusieurs colorants directs.

12. Composition selon la revendication 11, caractérisée par le fait que les colorants directs sont choisis dans le groupe formé par les colorants azoïques, les colorants anthraquinoniques et les dérivés nitrés de la série benzénique.

13. Composition selon les revendications 6 à 12, caractérisée par le fait qu'elle renferme de 0,02 à 6% en poids de composé de formule (I′) par rapport au poids total de la composition.

14. Composition selon les revendications 8 à 13, caractérisée par le fait que le poids total des précurseurs de colorants d'oxydation et des coupleurs est de 0,15 à 7% en poids du poids total de la composition tinctoriale.

15. Composition selon les revendications 6 à 14, caractérisée par le fait qu'elle comprend également des adjuvants choisis parmi les agents tensio-actifs anioniques, cationiques, non-ioniques, amphotères et leurs mélanges, les solvants organiques, les agents épaississants, les agents antioxydants, les agents de pénétration, les agents séquestrants, les tampons, les parfums, les agents alcalinisants.

16. Composition selon les revendications 6 à 15, caractérisée par le fait que son pH est de 8 à 11.

17. Procédé de teinture des cheveux humains, caractérisée par le fait qu'on applique sur les cheveux pendant 10 à 40 minutes et à une température comprise entre la température ambiante et 40°C, une quantité suffisante, pour teindre les cheveux, d'une composition selon l'une quelconque des revendications 6 à 16,

mélangée au moment de l'emploi avec une solution oxydante, en quantité suffisante pour oxyder les précurseurs de colorants d'oxydation, après quoi on rince les cheveux, on les lave au shampoing, on les rince à nouveau et on les sèche.

**18.** Procédé de teinture des cheveux en plusieurs étapes, caractérisé par le fait que :
(i) dans une première étape, on applique sur les cheveux une quantité suffisante pour les teindre, d'une composition selon l'une quelconque des revendications 6 à 8 et 11 à 16, contenant un ou plusieurs composés de formule (I'), éventuellement d'autres précurseurs de colorants d'oxydation; et
(ii) dans une deuxième étape, on applique sur les cheveux une solution contenant un ou plusieurs coupleurs, l'agent oxydant étant présent dans la composition appliquée dans la deuxième étape ou bien appliqué sur les cheveux eux-mêmes dans une troisième étape, des différentes compositions étant maintenues au contact des cheveux pendant 10 à 40 minutes à une température comprise entre la température ambiante et 40°C, après quoi on rince les cheveux, on les lave au shampooing, on les rince à nouveau et on les sèche.

## Claims

**Claims for the following Contracting States : AT, BE, CH, DE, FR, GB, GR, IT, LI, NL, SE**

**1.** 2-Substituted para-aminophenols of formula (I):

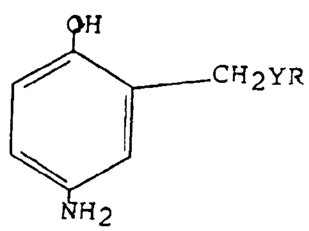

(I)

in which Y represents an oxygen or sulphur atom and R represents a $C_1$-$C_6$ alkyl radical or a $C_1$-$C_6$ hydroxyalkyl, $C_2$-$C_6$ polyhydroxyalkyl or $C_1$-$C_6$ halogenoalkyl radical, on condition that if Y denotes oxygen R is not methyl or ethyl and if Y denotes sulphur R is not ethyl, and their salts, except for 4-amino-2-[(2',2',2'-trifluoroethoxy)methyl]phenol.

**2.** 2-Substituted para-aminophenols according to Claim 1, characterised in that Y denotes a sulphur atom and R denotes a radical chosen from 2-hydroxyethyl, methyl and β,γ-dihydroxypropyl radicals.

**3.** 2-Substituted para-aminophenols according to Claim 1, characterised in that Y denotes an oxygen atom and R denotes the β-hydroxyethyl radical.

**4.** 2-Substituted para-aminophenols according to Claims 1 to 3, characterised in that they are in the form of the hydrochloride or the sulphate.

**5.** Tinctorial composition for dyeing keratinous fibres and in particular human hair, characterised in that it comprises, in an aqueous vehicle, one or more compounds of formula (I'):

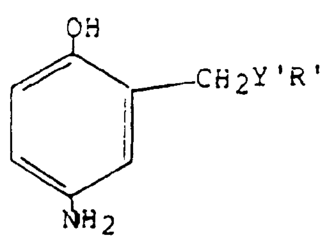

(I')

in which Y' represents an oxygen or sulphur atom and R' represents a $C_1$-$C_6$ alkyl, $C_1$-$C_6$ hydroxyalkyl, $C_2$-$C_6$ polyhydroxyalkyl or $C_1$-$C_6$ halogenoalkyl radical, or one or more salts of a compound of formula (I').

6. Composition according to Claim 5, characterised in that the compound of formula (I′) is chosen from the group formed by 4-amino-2-[(methylthio)methyl]phenol, 4-amino-2-[(β-hydroxyethylthio)methyl]phenol, 4-amino-2-methoxymethyl-phenol; 4-amino-2-ethoxymethyl-phenol, 4-amino-2-[(β-hydroxyethoxy)methyl]-phenol, 4-amino-2-[(β,γ-dihydroxypropylthio)methyl]-phenol and 4-amino-2-[(2′,2′,2′-trifluoroethoxy)methyl]-phenol.

7. Composition according to Claims 5 or 6, characterised in that it contains other oxidation dye precursors chosen from para-phenylenediamines, para-aminophenols other than those of formula (I′), ortho-phenylenediamines and ortho-aminophenols.

8. Composition according to Claims 5 to 7, characterised in that it also contains a coupler.

9. Composition according to Claim 8, characterised in that the coupler is chosen from the group formed by meta-diphenols, meta-aminophenols, meta-phenylenediamines, meta-acylaminophenols, meta-ureidophenols, meta-carbalkoxyaminophenols and alpha-naphthol, the couplers possessing an active methylene group, such as the β-ketone compounds and the pyrazolones, 3,4-methylenedioxyphenol, 3,4-methylenedioxyaniline, 2-bromo-4,5-methylenedioxyphenol, 2-chloro-4,5-methylenedioxyphenol and 2-methoxy-4,5-methylenedioxyaniline.

10. Composition according to Claims 5 to 9, characterised in that the composition also contains one or more direct dyes.

11. Composition according to Claim 10, characterised in that the direct dyes are chosen from the group formed by azo dyes, anthraquinone dyes and nitro derivatives of the benzene series.

12. Composition according to Claims 5 to 11, characterised in that it contains from 0.02 to 6% by weight of compound of formula (I′) with respect to the total weight of the composition.

13. Composition according to Claims 7 to 12, characterised in that the total weight of the oxidation dye precursors and the couplers is from 0.15 to 7% by weight of the total weight of the tinctorial composition.

14. Composition according to Claims 5 to 13, characterised in that it also contains adjuvants chosen from anionic, cationic, nonionic and amphoteric surfactants and their mixtures, organic solvents, thickeners, antioxidants, penetrating agents, sequestering agents, buffers, perfumes and alkalinising agents.

15. Composition according to Claims 5 to 14, characterised in that its pH is from 8 to 11.

16. Method for dyeing human hair, characterised in that a sufficient amount, for dyeing the hair, of a composition according to any one of Claims 5 to 15, mixed at the time of use with an oxidising solution, in an amount sufficient to oxidise the oxidation dye precursors, is applied to the hair for 10 to 40 minutes and at a temperature between ambient temperature and 40°C, after which the hair is rinsed, washed with a shampoo, rinsed again and dried.

17. Method for dyeing hair in several steps, characterised in that:
(i) in a first step, an amount, sufficient for dyeing the hair, of a composition according to any one of Claims 5 to 7 and 10 to 15, containing one or more compounds of formula (I′) and optionally other oxidation dye precursors is applied to the hair; and
(ii) in a second step, a solution containing one or more couplers is applied to the hair, the oxidising agent being present in the composition applied in the second step or being applied to the hair itself in a third step, the various compositions being kept in contact with the hair for 10 to 40 minutes at a temperature between ambient temperature and 40°C, after which the hair is rinsed, washed with shampoo, rinsed again and dried.

**Claims for the following Contracting State : ES**

1. Process for the preparation of 2-substituted para-aminophenols of formula (I):

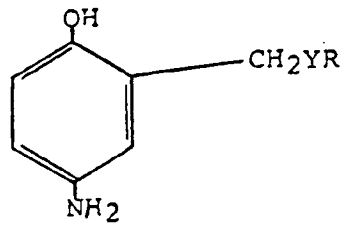

(I)

in which Y represents an oxygen or sulphur atom and R represents a $C_1$-$C_6$ alkyl radical or a $C_1$-$C_6$ hydroxyalkyl, $C_2$-$C_6$ polyhydroxyalkyl or $C_1$-$C_6$ halogenoalkyl radical, on condition that if Y denotes oxygen R is not methyl or ethyl and if Y denotes sulphur R is not ethyl, and their salts, except for 4-amino-2-[(2′,2′,2′-trifluoroethoxy)methyl]phenol, in accordance with the reaction scheme below:

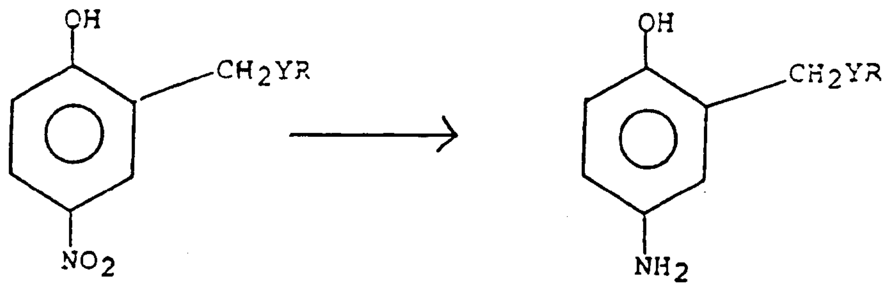

(II) (I)

by reduction of the compound of formula (II), where Y and R have the meanings indicated above.

2. Process according to Claim 1, characterised in that the reduction is carried out by means of sodium hydrosulphite in an alkaline medium at a temperature below 80°C, or by catalytic reduction in an aqueous-alcoholic medium, under hydrogen pressure, in the presence of a catalyst such as palladium-on-charcoal or nickel.

3. Preparation process according to Claim 1 or 2, characterised in that, in formula (I), Y denotes a sulphur atom and R denotes a radical chosen from 2-hydroxyethyl, methyl and β,γ-dihydroxypropyl radicals.

4. Preparation process according to Claims 1 or 2, characterised in that, in formula (I), Y denotes an oxygen atom and R denotes the β-hydroxyethyl radical.

5. Preparation process according to Claims 1 to 4, characterised in that the 2-substituted para-aminophenols of formula (I) are converted into an acid salt and preferably into the hydrochloride or sulphate.

6. Tinctorial composition for dyeing keratinous fibres and in particular human hair, characterised in that it comprises, in an aqueous vehicle, one or more compounds of formula (I′):

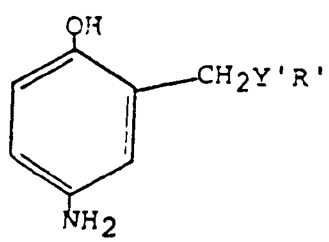

(I′)

in which Y′ represents an oxygen or sulphur atom and R′ represents a $C_1$-$C_6$ alkyl, $C_1$-$C_6$ hydroxyalkyl, $C_2$-$C_6$ polyhydroxyalkyl or $C_1$-$C_6$ halogenoalkyl radical, or one or more salts of a compound of formula (I′) obtained by a process according to Claims 1 to 5.

7. Composition according to Claim 6, characterised in that the compound of formula (I′) is chosen from the group formed by 4-amino-2-[(methylthio)methyl]phenol, 4-amino-2-[(β-hydroxyethylthio)methyl]phenol,

4-amino-2-methoxymethyl-phenol; 4-amino-2-ethoxymethyl-phenol, 4-amino-2-[(β-hydroxyethoxy)methyl]-phenol, 4-amino-2-[(β,γ-dihydroxypropylthio)methyl]-phenol and 4-amino-2-[(2′,2′,2′-trifluoroethoxy)methyl]-phenol.

8. Composition according to Claims 6 or 7, characterised in that it contains other oxidation dye precursors chosen from para-phenylenediamines, para-aminophenols other than those of formula (I′), ortho-phenylenediamines and ortho-aminophenols.

9. Composition according to Claims 6 to 8, characterised in that it also contains a coupler.

10. Composition according to Claim 9, characterised in that the coupler is chosen from the group formed by meta-diphenols, meta-aminophenols, meta-phenylenediamines, meta-acylaminophenols, meta-ureidophenols, meta-carbalkoxyaminophenols and alpha-naphthol, the couplers possessing an active methylene group, such as the β-ketone compounds and the pyrazolones, 3,4-methylenedioxyphenol, 3,4-methylenedioxyaniline, 2-bromo-4,5-methylenedioxyphenol, 2-chloro-4,5-methylenedioxyphenol and 2-methoxy-4,5-methylenedioxyaniline.

11. Composition according to Claims 6 to 10, characterised in that the composition also contains one or more direct dyes.

12. Composition according to Claim 11, characterised in that the direct dyes are chosen from the group formed by azo dyes, anthraquinone dyes and nitro derivatives of the benzene series.

13. Composition according to Claims 6 to 12, characterised in that it contains from 0.02 to 6% by weight of compound of formula (I′) with respect to the total weight of the composition.

14. Composition according to Claims 8 to 13, characterised in that the total weight of the oxidation dye precursors and the couplers is from 0.15 to 7% by weight of the total weight of the tinctorial composition.

15. Composition according to Claims 6 to 14, characterised in that it also contains adjuvants chosen from anionic, cationic, nonionic and amphoteric surfactants and their mixtures, organic solvents, thickeners, antioxidants, penetrating agents, sequestering agents, buffers, perfumes and alkalinising agents.

16. Composition according to Claims 6 to 15, characterised in that its pH is from 8 to 11.

17. Method for dyeing human hair, characterised in that a sufficient amount, for dyeing the hair, of a composition according to any one of Claims 6 to 16, mixed at the time of use with an oxidising solution, in an amount sufficient to oxidise the oxidation dye precursors, is applied to the hair for 10 to 40 minutes and at a temperature between ambient temperature and 40°C, after which the hair is rinsed, washed with a shampoo, rinsed again and dried.

18. Method for dyeing hair in several steps, characterised in that:
(i) in a first step, an amount, sufficient for dyeing the hair, of a composition according to any one of Claims 6 to 8 and 11 to 16, containing one or more compounds of formula (I′) and optionally other oxidation dye precursors is applied to the hair; and
(ii) in a second step, a solution containing one or more couplers is applied to the hair, the oxidising agent being present in the composition applied in the second step or being applied to the hair itself in a third step, the various compositions being kept in contact with the hair for 10 to 40 minutes at a temperature between ambient temperature and 40°C, after which the hair is rinsed, washed with shampoo, rinsed again and dried.

**Patentansprüche**

**Patentansprüche für folgende Vertragsstaaten : AT, BE, CH, DE, FR, GB, GR, IT, LI, NL, SE**

1. 2-Substituierte p-Aminophenole der Formel (I):

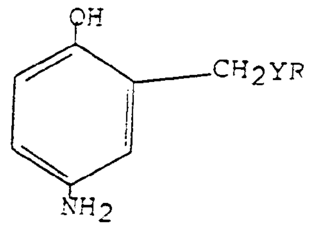

(I)

worin Y ein Sauerstoff- oder Schwefelatom und R einen $C_{1-6}$-Alkyl-, Hydroxy-$C_{1-6}$-alkyl-, Polyhydroxy-$C_{2-6}$-alkyl- oder Halogen-$C_{1-6}$-alkylrest darstellen, mit der Maßgabe, daß, wenn Y ein Sauerstoffatom bedeutet, R nicht Methyl oder Ethyl ist, und wenn Y ein Schwefelatom bedeutet, R nicht Ethyl ist, sowie deren Salze, ausgenommen 4-Amino-2-((2′,2′,2′-Trifluorethoxy)methyl)phenol.

2. 2-Substituierte p-Aminophenole gemäß Anspruch 1, dadurch **gekennzeichnet**, daß Y ein Schwefelatom und R einen Rest bedeuten, ausgewählt unter 2-Hydroxyethyl-, Methyl- und beta,gamma-Dihydroxypropylresten.

3. 2-Substituierte p-Aminophenole gemäß Anspruch 1, dadurch **gekennzeichnet**, daß Y ein Sauerstoffatom und R den beta-Hydroxyethylrest bedeuten.

4. 2-Substituierte p-Aminophenole gemäß Ansprüchen 1 bis 3, dadurch **gekennzeichnet**, daß sie in Form des Hydrochlorids oder Sulfats vorliegen.

5. Färbezusammensetzung zur Färbung keratinischer Fasern und insbesondere menschlicher Haare, dadurch **gekennzeichnet**, daß sie in einem wässrigen Träger eine oder mehrere Verbindungen der Formel (I′):

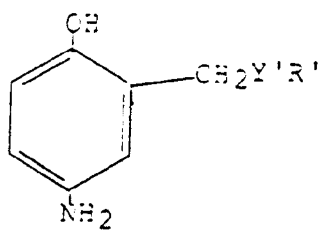

(I′)

worin Y′ ein Sauerstoff- oder Schwefelatom und R′ einen $C_{1-6}$-Alkyl-, Hydroxy-$C_{1-6}$-alkyl-, Polyhydroxy-$C_{2-6}$-alkyl- oder Halogen-$C_{1-6}$-alkylrest darstellen, oder ein Salz einer oder mehrerer Verbindungen der Formel (I′) enthält.

6. Zusammensetzung gemäß Anspruch 5, dadurch **gekennzeichnet**, daß die Verbindung der Formel (I′) ausgewählt ist aus der Gruppe aus 4-Amino-2-((methylthio)methyl)phenol, 4-Amino-2-((beta-hydroxyethylthio)methyl)phenol, 4-Amino-2-methoxymethylphenol, 4-Amino-2-ethoxymethylphenol, 4-Amino-2-((beta-hydroxyethoxy)methyl)phenol, 4-Amino-2-((beta,gamma-dihydroxypropylthio)methyl)-phenol und 4-Amino-2-((2′,2′,2′-trifluorethoxy)methyl)phenol.

7. Zusammensetzung gemäß der Ansprüche 5 oder 6, dadurch **gekennzeichnet**, daß sie weitere Oxidationsfarbstoff-Vorstufen enthält, ausgewählt aus p-Phenylendiaminen, von denjenigen der Formel (I′) verschiedenen p-Aminophenolen, o-phenylendiaminen und o-Aminophenolen.

8. Zusammenetzung gemäß Ansprüchen 5 bis 7, dadurch **gekennzeichnet**, daß sie auch einen Kuppler enthält.

9. Zusammensetzung gemäß Anspruch 8, dadurch **gekennzeichnet**, daß der Kuppler ausgewählt ist aus der Gruppe aus m-Diphenolen, m-Aminophenolen, m-Phenylendiaminen, m-Acylaminophenolen, m-Ureidophenolen, m-Carbalkoxyaminophenolen, alpha-Naphthol, Kupplern mit einer aktiven Methylengruppe wie den beta-Ketoverbindungen und Pyrazolonen, 3,4-Methylendioxyphenol, 3,4-Methylendioxyanilin, 2-Brom-4,5-methylendioxyphenol, 2-Chlor-4,5-methylendioxyphenol und 2-Methoxy-4,5-methylendioxyanilin.

10. Zusammensetzung gemäß der Ansprüche 5 bis 9, dadurch **gekennzeichnet**, daß die Zusammensetzung

auch einen oder mehrere Direkt-Farbstoffe enthält.

11. Zusammensetzung gemäß Anspruch 10, dadurch **gekennzeichnet**, daß die Direkt-Farbstoffe ausgewählt sind aus der Gruppe aus Azo-, Anthrachinonfarbstoffen und den nitrierten Derivaten der Benzolreihe.

12. Zusammensetzung gemäß der Ansprüche 5 bis 11, dadurch **gekennzeichnet**, daß sie 0,02 bis 6 Gew.% der Verbindung der Formel (I'), bezogen auf Gesamtgewicht der Zusammensetzung, enthält.

13. Zusammensetzung gemäß der Ansprüche 7 bis 12, dadurch **gekennzeichnet**, daß das Gesamtgewicht der Oxidationsfarbstoff-Vorstufen und Kuppler 0,15 bis 7 Gew.% des Gesamtgewichts der Färbezusammensetzung beträgt.

14. Zusammensetzung gemäß der Ansprüche 5 bis 13, dadurch **gekennzeichnet**, daß sie auch Hilfstoffe enthält, ausgewählt aus anionischen, kationischen, nicht-ionischen, amphoteren oberflächenaktiven Mitteln und deren Mischungen, organischen Lösungsmitteln, Verdickungsmitteln, Antioxidantien, Penetrationsmitteln, Sequestriermitteln, Tampons, Parfüms, alkalisch machenden Mitteln.

15. Zusammensetzung gemäß der Ansprüche 5 bis 14, dadurch **gekennzeichnet**, daß ihr pH 8 bis 11 beträgt.

16. Verfahren zur Färbung menschlicher Haare, dadurch **gekennzeichnet**, daß man auf die Haare während 10 bis 40 Minuten bei einer Temperatur von Umgebungstemperatur bis 40°C eine zur Färbung der Haare hinreichende Menge einer Zusammensetzung gemäß jedem der Ansprüche 5 bis 15 aufbringt, die zum Zeitpunkt des Gebrauchs mit einer oxidierenden Lösung in einer zum Oxidieren der Oxidationsfarbstoff-Vorstufen hinreichenden Menge vermischt wird, worauf man die Haare spült, unter Schamponieren wäscht, erneut spült und trocknet.

17. Verfahren zum Färben der Haare in mehreren Stufen, dadurch **gekennzeichnet**, daß man: (i) in einer ersten Stufe auf die Haare eine zum Färben hinreichende Menge einer Zusammensetzung gemäß jedem der Ansprüche 5 bis 7 und 10 bis 15, enthaltend einen oder mehrere Verbindungen der Formel (I') und gegebenenfalls weitere Oxidationsfarbstoff-Vorstufen, aufbringt und (ii) in einer zweiten Stufe auf die Haare eine Lösung, enthaltend einen oder mehrere Kuppler, aufträgt, wobei das Oxidationsmittel in der in der zweiten Stufe aufgebrachten Zusammensetzung vorhanden ist oder auch auf die Haare selbst in einer dritten Stufe aufgebracht wird, und wobei die verschiedenen Zusammensetzungen in Kontakt mit den Haaren während 10 bis 40 Minuten bei einer Temperatur von Umgebungstemperatur bis 40°C gehalten werden, worauf man die Haare spült, unter Schamponieren wäscht, erneut spült und trocknet.

**Patentansprüche für folgenden Vertragsstaat : ES**

1. Verfahren zur Herstellung von 2-substituierten p-Aminophenolen der Formel (I):

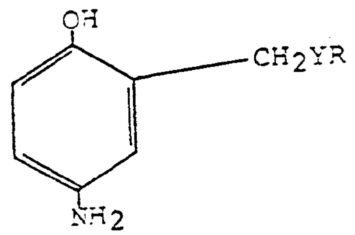

(I)

worin Y ein Sauerstoff- oder Schwefelatom und R einen $C_{1-6}$-Alkyl-, Hydroxy-$C_{1-6}$-alkyl-, Polyhydroxy-$C_{2-6}$-alkyl- oder Halogen-$C_{1-6}$-alkylrest darstellen, mit der Maßgabe, daß, wenn Y ein Sauerstoffatom bedeutet, R nicht Methyl oder Ethyl ist, und wenn Y ein Schwefelatom bedeutet, R nicht Ethyl ist, sowie deren Salze, ausgenommen 4-Amino-2-((2',2',2'-Trifluorethoxy)methyl)phenol, gemäß des nachfolgenden Reaktionsschemas:

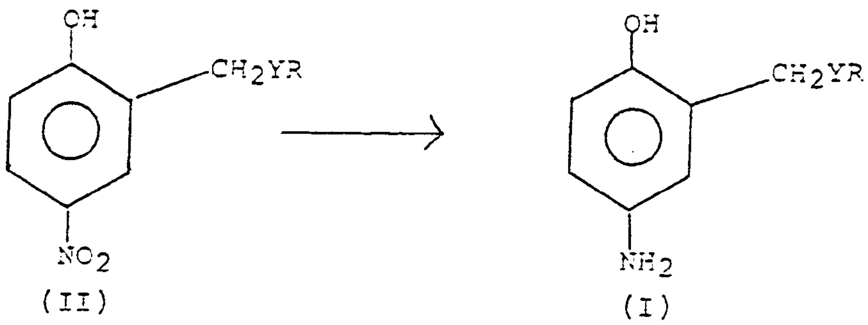

und zwar durch Reduktion der Verbindung der Formel (II), worin Y und R die oben angegebenen Bedeutungen haben.

**2.** Verfahren gemäß Anspruch 1, dadurch **gekennzeichnet**, daß die Reduktion mit Natriumhydrogensulfit in alkalischem Milieu bei einer Temperatur unterhalb 80°C oder auch durch katalytische Reduktion in hydroalkoholischem Milieu, unter Wasserstoffdruck, in Gegenwart eines Katalysators wie Palladium auf Kohle oder wie Nickel durchgeführt wird.

**3.** Verfahren zur Herstellung gemäß Anspruch 1 oder 2, dadurch **gekennzeichnet**, daß in der Formel (I) Y ein Schwefelatom und R einen Rest bedeuten, ausgewählt unter 2-Hydroxyethyl-, Methyl- und beta,gamma-Dihydroxypropylresten.

**4.** Verfahren zur Herstellung gemäß der Ansprüche 1 oder 2, dadurch **gekennzeichnet**, daß in der Formel (I) Y ein Sauerstoffatom und R den beta-Hydroxyethylrest bedeuten.

**5.** Verfahren zur Herstellung gemäß der Ansprüche 1 bis 4, dadurch **gekennzeichnet**, daß die 2-substituierten p-Aminophenole der Formel (I) in ein Säuresalz, vorzugsweise ein Hydrochlorid oder Sulfat, überführt werden.

**6.** Färbezusammensetzung zur Färbung keratinischer Fasern und insbesondere menschlicher Haare, dadurch **gekennzeichnet**, daß sie in einem wässrigen Träger eine oder mehrere Verbindungen der Formel (I′):

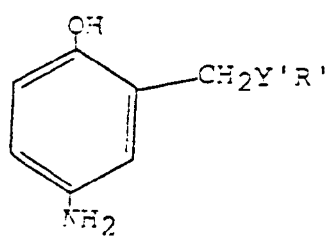

(I′)

worin Y′ ein Sauerstoff- oder Schwefelatom und R′ einen $C_{1-6}$-Alkyl-, Hydroxy-$C_{1-6}$-alkyl-, Polyhydroxy-$C_{2-6}$-alkyl- oder Halogen-$C_{1-6}$-alkylrest darstellen, oder ein Salz einer oder mehrerer Verbindungen der Formel (I′) enthält, erhältlich durch ein Verfahren gemäß der Ansprüche 1 bis 5.

**7.** Zusammensetzung gemäß Anspruch 6, dadurch **gekennzeichnet**, daß die Verbindung der Formel (I′) ausgewählt ist aus der Gruppe aus 4-Amino-2-((methylthio)methyl)phenol, 4-Amino-2-((beta-hydroxyethylthio)methyl)phenol, 4-Amino-2-methoxymethylphenol, 4-Amino-2-ethoxymethylphenol, 4-Amino-2-((beta-hydroxyethoxy)methyl)phenol, 4-Amino-2-((beta,gamma-dihydroxypropylthio)methyl)-phenol, und 4-Amino-2-((2′,2′,2′-trifluorethoxy)methyl)phenol.

**8.** Zusammensetzung gemäß der Ansprüche 6 oder 7, dadurch **gekennzeichnet**, daß sie weitere Oxidationsfarbstoff-Vorstufen enthält, ausgewählt aus p-Phenylendiaminen, von denjenigen der Formel (I′) verschiedenen p-Aminophenolen, o-Phenylendiaminen und o-Aminophenolen.

**9.** Zusammenetzung gemäß Ansprüchen 6 bis 8, dadurch **gekennzeichnet**, daß sie auch einen Kuppler enthält.

10. Zusammensetzung gemäß Anspruch 9, dadurch **gekennzeichnet**, daß der Kuppler ausgewählt ist aus der Gruppe aus m-Diphenolen, m-Aminophenolen, m-Phenylendiaminen, m-Acylaminophenolen, m-Ureidophenolen, m-Carbalkoxyaminophenolen, alpha-Naphthol, Kupplern mit einer aktiven Methylengruppe wie den beta-Ketoverbindungen und Pyrazolonen, 3,4-Methylendioxyphenol, 3,4-Methylendioxyanilin, 2-Brom-4,5-methylendioxyphenol, 2-Chlor-4,5-methylendioxyphenol und 2-Methoxy-4,5-methylendioxyanilin.

11. Zusammensetzung gemäß der Ansprüche 6 bis 10, dadurch **gekennzeichnet**, daß die Zusammensetzung auch einen oder mehrere Direkt-Farbstoffe enthält.

12. Zusammensetzung gemäß Anspruch 11, dadurch **gekennzeichnet**, daß die Direkt-Farbstoffe ausgewählt sind aus der Gruppe aus Azo-, Anthrachinonfarbstoffen und den nitrierten Derivaten der Benzolreihe.

13. Zusammensetzung gemäß der Ansprüche 6 bis 12, dadurch **gekennzeichnet**, daß sie 0,02 bis 6 Gew.% der Verbindung der Formel (I′), bezogen auf Gesamtgewicht der Zusammensetzung, enthält.

14. Zusammensetzung gemäß der Ansprüche 8 bis 13, dadurch **gekennzeichnet**, daß das Gesamtgewicht der Oxidationsfarbstoff-Vorstufen und Kuppler 0,15 bis 7 Gew.% des Gesamtgewichts der Färbezusammensetzung beträgt.

15. Zusammensetzung gemäß der Ansprüche 6 bis 14, dadurch **gekennzeichnet**, daß sie auch Hilfstoffe enthält, ausgewählt aus anionischen, kationischen, nicht-ionischen, amphoteren oberflächenaktiven Mitteln und deren Mischungen, organischen Lösungsmitteln, Verdickungsmitteln, Antioxidantien, Penetrationsmitteln, Sequestriermitteln, Tampons, Parfüms, alkalisch machenden Mitteln.

16. Zusammensetzung gemäß der Ansprüche 6 bis 15, dadurch **gekennzeichnet**, daß ihr pH 8 bis 11 beträgt.

17. Verfahren zur Färbung menschlicher Haare, dadurch **gekennzeichnet**, daß man auf die Haare während 10 bis 40 Minuten bei einer Temperatur von Umgebungstemperatur bis 40°C eine zur Färbung der Haare hinreichende Menge einer Zusammensetzung gemäß jedem der Ansprüche 6 bis 16 aufbringt, die zum Zeitpunkt des Gebrauchs mit einer oxidierenden Lösung in einer zum Oxidieren der Oxidationsfarbstoff-Vorstufen hinreichenden Menge vermischt wird, worauf man die Haare spült, unter Schamponieren wäscht, erneut spült und trocknet.

18. Verfahren zum Färben der Haare in mehreren Stufen, dadurch **gekennzeichnet**, daß man: (i) in einer ersten Stufe auf die Haare eine zum Färben hinreichende Menge einer Zusammensetzung gemäß jedem der Ansprüche 6 bis 8 und 11 bis 16, enthaltend eine oder mehrere Verbindungen der Formel (I′) und gegebenenfalls weitere Oxidationsfarbstoff-Vorstufen, aufbringt und (ii) in einer zweiten Stufe auf die Haare eine Lösung, enthaltend einen oder mehrere Kuppler, aufträgt, wobei das Oxidationsmittel in der in der zweiten Stufe aufgebrachten Zusammensetzung vorhanden ist oder auch auf die Haare selbst in einer dritten Stufe aufgebracht wird, und wobei die verschiedenen Zusammensetzungen in Kontakt mit den Haaren während 10 bis 40 Minuten bei einer Temperatur von Umgebungstemperatur bis 40°C gehalten werden, worauf man die Haare spült, unter Schamponieren wäscht, erneut spült und trocknet.